# EUROPEAN PATENT SPECIFICATION

(11) **EP 1 556 494 B1**
(45) Date of publication and mention of the grant of the patent: **01.08.2007**
(21) Application number: 03754321.2
(22) Date of filing: 05.08.2003
(51) Int. Cl.: C12N 15/62, A61K 48/00, A61P 9/10

(54) **BICISTRONIC VECTOR ENCODING A VEGF AND AN FGF, USE THEREOF**
BICISTRONISCHER VEKTOR DER EIN VEGF UND EIN FGF ENKODIERT, VERWENDUNG DAVON
VECTEUR BICISTRONIQUE QUI ENCODE UN VEGF ET UN FGF, SON UTILISATION

(30) Priority: 05.08.2002 PL 35535402
(43) Date of publication of application: 27.07.2005
(73) Proprietor: Zaklady Farmaceutyczne "Polpharma" S.A., PL-83-200 Starogard Gdanski (PL)
(72) Inventor: MALECKI, Maciej, PL-05-500 Piaseczno (PL); JANIK, Przemyslaw, PL-01-571 Warszawa (PL); PRZYBYSZEWSKA, Malgorzata, PL-02-781 Warszawa (PL); MOSTOWSKA-WAS, Irena, PL-05-090 Raszyn (PL)
(74) Representative: Witek, Rafal
(86) International application number: PCT/PL2003/000076
(87) International publication number: WO 2004/012775

(56) References cited:
- EP-A- 1 132 479
- WO-A-00/24412
- WO-A-00/31284
- WO-A-00/53790
- WO-A-01/12830
- WO-A-01/34208
- WO-A-01/81544
- WO-A-97/14307
- LAI ET AL.: "The Construction and Expression of Polycistronic Retroviral Vector Carrying Genes of TNF-alpha, IL-2 and NeoR" , [Online] XP002270387 Retrieved from the Internet: <URL:http://www.ncbi.nlm.nih.gov:80/entrez /query.fcgi?cmd=Retrieve&db=PubMed&list_ui ds=12174276&dopt=Abstract> [retrieved on 2004-02-16] & SHENG WU HUA XUE YU SHENG WU WU LI XUE BAO (SHANGHAI), vol. 30, no. 2, 1998, pages 159-163,
- DATABASE BIOSIS [Online] BIOSCIENCES INFORMATION SERVICE, PHILADELPHIA, PA, US; March 2000 (2000-03) TANG ZHANYUN ET AL: "Construction of polycistronic retroviral vector containing gene of mIL-12 and the expression in murine hepatoma cells" Database accession no. PREV200000191080 XP002270388 & ZHONGHUA WEISHENGWUXUE HE MIANYIXUE ZAZHI, vol. 20, no. 2, March 2000 (2000-03), pages 93-97, ISSN: 0254-5101
- DATABASE BIOSIS [Online] BIOSCIENCES INFORMATION SERVICE, PHILADELPHIA, PA, US; 2003 MALECKI MACIEJ ET AL: "Construction of a bicistronic proangiogenic expression vector and its application in experimental angiogenesis in vivo." Database accession no. PREV200400081422 XP002270389 & ACTA BIOCHIMICA POLONICA, vol. 50, no. 3, 2003, pages 875-882, ISSN: 0001-527X

## Description

### FIELD OF THE INVENTION

The present invention relates to an expression box, two-cistron plasmid vector, pharmaceutical composition and their application in angiogenic gene therapy.

### BACKGROUND OF THE INVENTION

The therapeutic application of angiogenic growth factors was described for the first time by Folkman et al. (Folkman, N Engl J Med, 285:1182-1186 (1971)). Later studies have confirmed the utility of recombinant angiogenic growth factors, such as the fibroblast growth factor family (FGF) (Yanagisawa-Miwa, et al., Science, 257:1401-1403 (1992) i Baffour, et al., J Vasc Surg, 16:181-91 (1992)), endohelial cell growth factor (ECGF)(Pu, et al., J Surg Res, 54:575-83 (1993)), as well as the vascular endothelial growth factor (VEGF) in the stimulation of development and maturation of new blood vessels in an animal model of ischemia, particularly in cardiac muscle (Takeshita, et al., Circulation, 90:228-234 (1994) and Takeshita, et al., J Clin Invest, 93:662-70 (1994)). In the mentioned studies, recombinant proteins were applied intramuscularly. The significant limitation of this type of therapy are the difficulties in maintaining the correct concentration of growth factors at the treatment site.

An alternative method for recombinant protein treatment is the application of gene therapy, which consists of the insertion of DNA coding the factors in question into the affected site. Many attempts have been made to utilise sequences coding angiogenic growth factors in therapy.

Patent application WO 97/14307 pertains to a method of treatment of ischemia in a mammal, which consists of injecting into the said tissue of an effective quantity of nucleic acid capable of the expression of the angiogenic protein. The method of the present invention may be applied in the treatment of any ischemic tissue, that is a tissue defficient in blood supply as a result of ischemia. Examples of these tissue are muscles (including cardiac muscle), the brain, kidneys and lungs. A plasmid has been described containing the cDNA of human VEGF-165 under control of the CMV promoter. On page 9, paragraph 3, it has been stated that "a beneficial angiogenic protein contains a secretory signal sequence which facilitates the secretion of the protein. Also of benefit are angiogenic proteins possessing native signal sequences, i.e. VEGF. Angiogenic proteins without a native signal sequence, i.e. bFGF, may be modified using known methods, so that they may possess such a sequence (...)" The problem posed in this way does not take into account the possibility of replacing the native secretory sequence of VEGF-165 with another known sequence, i.e. in order to improve secretion, and suggests that the most appropriate sequences are those natively appearing in the sequence of a given protein.

Animal tests were described (rabbits and calves), which presented qualitative and quantitative evidence of the attainment of the desirable physiological effect. There are no data reporting human therapy.

Patent application WO 97/12519 pertains to a method of the re-endothelisation damaged blood vessels with gene therapy. A plasmid containing the cDNA of human VEGF-165 under the control of the CMV promoter was applied. The expression box came from phVEGF-165 described in WO 97/14307 (above). The description also contains the same statements regarding the secretory sequence.

Animal tests were described (rabbits and calves), which presented qualitative and quantitative evidence of the attainment of the desirable physiological effect. There are no data reporting human therapy.

Patent application WO 00/24412 pertains to products and methods of prevention of the constriction or re-constriction of vessels using the VEGF-C and/or VEGF-D genes and proteins.

On page 7 of the description (lines 8-19) the authors demonstrated possible changes to the VEGF-C sequence used, and at the same time they underlined the importance of the secretory sequence in the proper secretion of the planned proteins. They suggested the application of a secretory sequence from VEGF-C along with the described derivatives of this protein. It was also suggested to change the native secretory sequence for another secretory sequence.

Patent application WO 01/34208 pertains to the treatment of patients with circulatory illness, including heart disease and peripheral circulatory system disorders. The methods under the invention include the *in vivo* administration of genes coding angiogenic peptides or proteins into heart muscle or peripheral tissue affected with poor blood flow, through the introduction of a vector containing a gene into blood vessels supplying the heart or into the ischemic tissue. Example 7 reveals the results of an experiment whose aim was to increase the level of the angiogenic protein FGF-2 possessing no native expression sequence. The desired effect (an increase in the stimulation of angiogenesis) was achieved by concatenating the secretory sequence of FGF-4. It was suggested that the sequence added should stem from a protein expressed in cardiac muscle cells. It was also suggested that it may also be beneficial to utilise the FGF-4 and VEGF-145 genes in parallel, which according to the authors may lead to a synergistic effect No evidence was given, however, to support this conjecture. Only animal tests were described. There are no data describing the therapy in humans.

### THE GOAL OF THE INVENTION

In the light of the current state of-knowledge there still remains a need for therapeutic agents which could be used in gene therapy of circulatory illness in humans. Problems which need to be solved involve the efficacy of the administered medicines, for example through increasing the level of secretion of the expressed angiogenic factors. Other problems in need of solution are the quality and durability of the induced vessels, which should reach full maturity in order to improve the therapeutic effect. It would also be beneficial to limit the potential toxicity of injection mixtures containing the therapeutic DNA, as well as to lower their cost.

The problems indicated were solved in the present invention.

### SUMMARY OF THE INVENTION

The present invention provides an expression box consisting of a CMV promoter, and the polyadenylation signal sequence SV40, connected operationally with a gene complex containing:
a) a sequence coding a hybrid protein containing a secretory sequence stemming from immunoglobulin bound to the vascular endothelial growth factor 165 (VEGF₁₆₅) sequence with a removed native secretory sequence,
b) a sequence coding the hybrid protein containing a secretory sequence stemming from immunoglobulin bound to the fibroblast growth factor b (bFGF) sequence,
c) an Internal Ribosomal Entry Segment (IRES) sequence located between the mentioned sequences coding hybrid proteins,
wherein said secretory sequence stemming from immunoglobulin comprises amino acids residues 1-40 of SEQ ID No.:1.

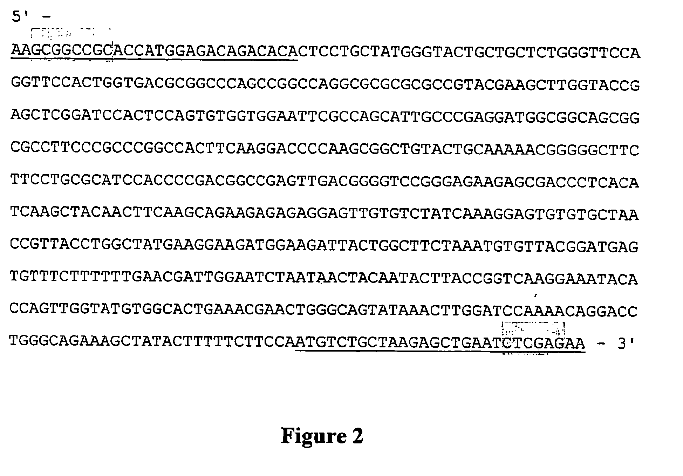

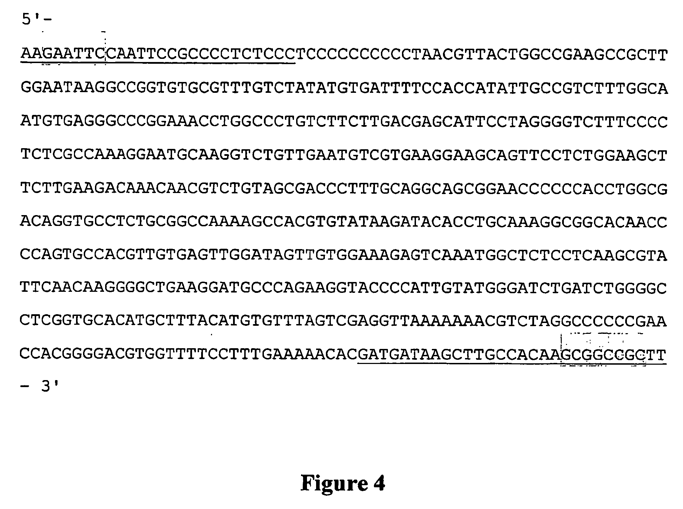

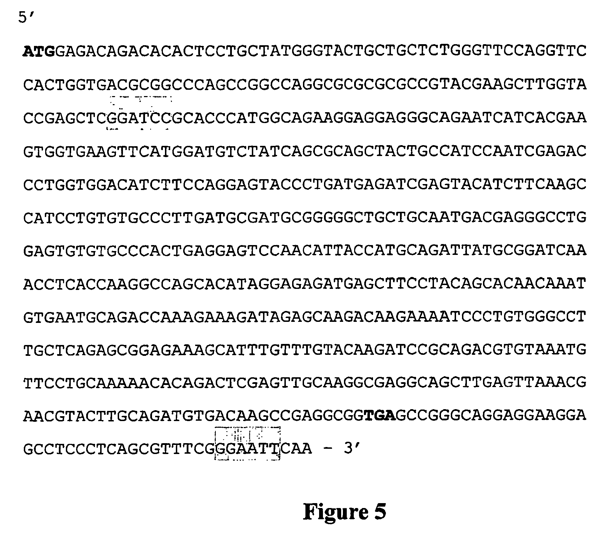

Possibly, the sequence coding the hybrid protein mentioned in (a) is the coding sequence shown in fig. 5 or sequence presented as SEQ ID No.:1, and the sequence coding the hybrid protein mentioned in (b) is the sequence shown in fig. 2 or sequence presented as SEQ ID No.:3, and if the IRES sequence mentioned in (c) is the sequence presented in Figure 4 or nucleotides 1348-1953 of SEQ ID No.:6. Preferably, the expression box according to the invention contains at least the nucleotides from 695 to 2538 of sequence presented as SEQ ID No.:5.

A further aspect of the invention is a two-cistron plasmid vector, characterised by the fact that it contains an expression box according to the invention. Here, possibly this plasmid vector is pVIF.

Another aspect of the invention is the application of the expression box according to the invention for the production of an expression vector for the gene therapy of circulatory deficiency in humans. In particular, the topic of the invention is the application of the pVIF plasmid in the production of a treatment for circulatory deficiency in humans.

The next aspect of the invention is a pharmaceutical composition for the treatment of circulatory deficiency in humans which contains an active ingredient and a pharmaceutically acceptable diluent, characterised by the fact that as the active ingredient it contains a two-cistron plasmid vector containing an expression box according to the invention. Here, the active ingredient is the pVIF plasmid.

Another aspect of the invention is also a use of the expression box or vector according to the invention, as defined above, for the production of an medication for the treatment or prevention of ischemic disorders or diseases of the human being. Such medication can be administered either in or nearby to site of the ischemic tissue. Here, ischemic tissue is an ischemic myocardium.

Another aspect of the invention is also a use of the expression box or vector according to the invention, as defined above, for the production of an medication for revascularizing ischemic tissue or improving the vascular flow of ischemic tissue. Such medication can be administered either in or nearby to site of the ischemic tissue. Here, ischemic tissue is an ischemic myocardium.

Medications in accordance with the invention may be administered intramuscularly or intravenously. For example, in the case of cardiac muscle ischemia intramuscular administration is possible, as is administration into the correct area of the cardiac capillaries.

### THE VIRTUES OF THE INVENTION

The present invention unexpectedly facilitates a significant increase in the effectiveness of angiogenic gene therapy. Unexpectedly, using cross-species secretory sequences, an increase in the level of secretion of angiogenic factors was achieved. Unexpectedly, the presented vector induces the production of more mature and stabilised blood vessels *in vivo,* in comparison to a vector coding a single factor, i.e. only VEGF, and when applied in gene therapy (trials on patients) it causes more effective and faster improvements in the clinical state of the patient. An injection of a two-cistron vector entails a lesser introduction of foreign

DNA as well as endotoxins, which may be contaminating the DNA preparation, than in the case of the administration of two single-gene vectors. By the same token one nullifies the danger of toxicity induced by the medication or other serious complications (fevers, shock and unconsciousness). Not meaningless are also the lower material costs needed for the production of one two-cistron vector. It is evident that the cost of isolation, purification, and endotoxin level testing is smaller than that of two single-gene vectors. Thanks to this the process of production of means may be more efficient with the same expenditure. This increases the availability of the medication.

The following examples will illustrate the invention further.

The recombinant DNA methods employed in practising the present invention are standard procedures, well known to those skilled in the art, and described in detail, for example, in "Molecular Cloning" (Sambrook et al. 1989) and in "A Practical Guide to Molecular Cloning" (Perbal, 1984).

### BRIEF DESCRIPTION OF THE DRAWINGS

Figure 1 shows a schematic of the expression box of the pVIF vector. The following acronyms were used: CMV- Cytomegaly virus promoter, ATG - start codon, TGA - stop codon, Igκ - mouse immunoglobulin signal sequence, VEGF - gene coding for the vascular endothelial growth factor, bFGF - gene coding the basic fibroblast growth factor, IRES - internal ribosome entry site.
Figure 2 shows the sequence of the ATG-Igκ-bFGF cloned into a pSEC/VEGF/IRES vector (NotI-ATG-Igκ-bFGF-XhoI). The primers used are underlined, the restriction sites used are shaded.

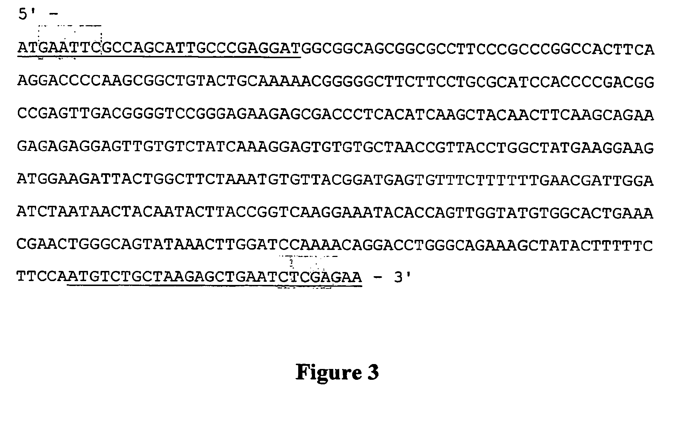

Figure 3 shows the bFGF sequence cloned into a pSEC (EcoRI-bFGF-XhoI) vector. The primers used are underlined.
Figure 4 shows the IRES sequence cloned into a pSEC/VEGF (EcoRI-IRES-NotI) vector. RBS-1 and RBS-2 primers are underlined, restriction sites used (EcoRI, Not I) are shaded.
Figure 5 shows the sequence ATG-Igκ-VEGF cloned into a pSEC/VEGF vector. Start and stop codons are in bold, fragments coding restriction sites are shaded.

For a better understanding of the essence of the invention, it is illustrated below through examples.

### Example 1. The pVIF plasmid.

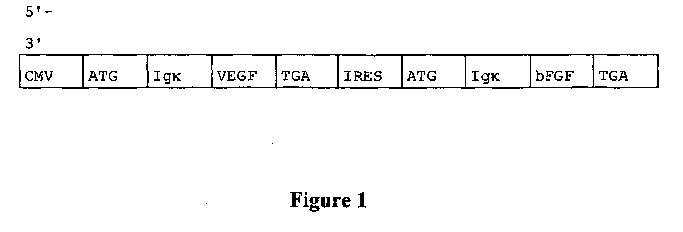

The pVIF construct is a plasmid expression vector coding angiogen-type factors. The cloned-in box contains two cistrons. It contains a gene coding VEGF 165 (PubMed X62568), the IRES sequence (based on a template cloned onto a pIRES-EGFP vector, Clontech, cat. #6064-1) as well as a sequence coding bFGF (Pubmed E02544). The IRES sequence stems from the encephalomyocarditis virus (ECMV) and ensures the translation of both cloned genes from one two-cistron mRNA. Both the VEGF and the FGF sequences are preceded by signal sequences in the box, which facilitate the secretion of the synthesized VEGF and FGF proteins. Figure 1 shows a schematic of the expression box in the pVIF vector.

### Example 2. Obtaining the pVIF vector.

Cloning was carried out using sticky ends to the ATG triplet in accordance with the reading frame. Amersham reagents were used, and the vector was amplified in *E.coli* bacteria, of the DH5α strain. The isolation was carried out using the EndoFree Plasmid Mega Kit from Qiagen.

To obtain the pVIF vector, two supplementary vectors were used, pSEC/VEGF as well as an empty pSEC vector. The pSEC/VEGF vector was cloned earlier according to the description contained in Polish patent application P.339326, and it is a plasmid expression vector coding the VEGF165 isoform. It contains a signal sequence facilitating the secretion of the VEGF protein outside of the cell. The procedure was composed of three steps: (a) subcloning of the IRES sequence from the pMIG/IRES vector to a pSEC/VEGF vector, (b) cloning of the bFGF sequence onto a pSEC vector, (c) subcloning of the ATG - Igκ - bFGF box from the pSEC/bFGF vector to the pSEC/VEGF/IRES vector.
A. Subcloning of the IRES sequence from the pMIG/IRES vector to a pSEC/VEGF vector. The IRES sequence was amplified using PCR from a template sequence cloned into a pMIG wector. The PCR primers were designed to contain the restriction sites for EcoRI and NotI:
   RBS-1: 5' - AAGAATTCCAATTCCGCCCCTCTCCCT - 3'
   RBS-2: 5' - AAGCGGCCGCTTGTGGCAAGCTTATCATC - 3'

   The PCR product was purified using the Clean-Up kit (DNA-Gdańsk), digested with EcoRI and NotI for 1 h at a temp. of 37 °C and isolated from an agarose gel using a QIAquick Gel Extraction Kit from Qiagen. The pSEC/VEGF vector was also digested with EcoRI and NotI, separated electrophoretically and isolated from the gel. Ligation was carried out over 16 h at 16 °C using T4 ligase (Amersham). Ligants were transformed into *E.coli* bacteria, and a clone selection was made on agar dishes with ampicillin. Grown clones were transferred to a liquid LB medium with amipicillin. They were incubated overnight at 37°C. Plasmids were isolated from the clones, which were then restriction-analysed. Thus the pSEC/VEGF/IRES vector was obtained.
B. Cloning of the bFGF sequence onto the pSEC vector
   The sequence coding bFGF was amplified using a template of cDNA from cells of the HUVEC line (human urolimbical vein endothelial cell). The PCR primers were designed to contain the restriction sites for EcoRI and XhoI:
   HbF-1: 5' - ATGAATTCGCCAGCATTGCCCGAGGAT - 3'
   HbF-2: 5' - TTCTCGAGATTCAGCTCTTAGCAGACAT - 3'

   The PCR product was purified using the Clean-Up kit (DNA-Gdańsk), digested with EcoRI and XhoI for 1 h at a temp. of 37 °C and isolated from an agarose gel using a QIAquick Gel Extraction Kit from Qiagen. The pSEC vector was also digested with EcoRI and XhoI, separated electrophoretically and isolated from the gel. Ligation was carried out over 16 h at 16 °C using T4 ligase (Amersham). Ligants were transformed into *E. coli* bacteria, and a clone selection was made on agar dishes with ampicillin. Grown clones were transferred to a liquid LB medium with amipicillin. They were incubated overnight at 37°C. Plasmids were isolated from the clones, which were then restriction-analysed. Thus the pSEC/FGF vector was obtained.
C. Subcloning of the ATG - Igκ- bFGF box from the pSEC/bFGF vector onto the pSEC/VEGF/IRES vector
   The ATG - Igκ- FGF box was cloned from the pSEC/FGF vector onto the pSEC/VEGF/IRES vector. This fragment was amplified using PCR. A new primer was designed, which introduced a NotI restriction site, and the HbF-2 primer was also used. The sequence of the sense primer was as follows:
   Not_IgK: 5' - AAGCGGCCGCACCATGGAGACAGACACA - 3'

From then, the procedure was carried out as before:: the PCR product was purified using the Clean-Up kit (DNA-Gdańsk), digested with NotI and XhoI over 1 h at a temp. of 37 °C and isolated from an agarose gel using the QIAquick Gel Extraction Kit from Qiagen. The pSEC/VEGF/IRES vector was also digested with NotI and XhoI, separated electrophoretically and isolated from the gel. Ligation was carried out over 16 h at 16 °C using T4 ligase (Amersham). Ligants were transformed into *E.coli* bacteria, and a clone selection was made on agar dishes with ampicillin. Grown clones were transferred to a liquid LB medium with amipicillin. They were incubated overnight at 37°C. Plasmids were isolated from the clones, which were then restriction-analysed. Thus the pSEC/VEGF/IRES/FGF (pVIF) vector was obtained.

The construct obtained underwent a detailed restriction analysis. Various combinations of restrictases were used, as were several PCRs using various primers. The construct was also sequenced. All analyses confirmed the correct cloning. A vector with a two-cistron VEGF/IRES/FGF expression box was obtained.

### Example 3. In vitro assays

The constructed pVIF vector was inserted into tumour cells (LI line - murine sarcoma) and non-tumour cells (HCV-29 line - human urinary bladder epithelium) each with a low expression of VEGF and FGF growth factors. Using Western blotting, using specific monoclonal anti-VEGF and anti-FGF antibodies, we analysed the presence of these proteins in the medium surrounding the transfected cells. The analysis confirmed the expression of genes borne by the pVIF vector in the transfected cells, and that the synthesised VEGF and FGF proteins are secreted to the outside of the cell.

### Example 4. In vivo studies

The studies were carried out in BALB/c strain mice (males). A skin angiogenesis test was performed. The mice received the vector subcutaneously at a dose of 10 µg per site. After 3 and 13 days following the injection new blood vessels were counted. It was concluded that the pVIF vector clearly induces the formation of blood vessels. The results were compiled in table 1.

**Table 1. Comparison of effects**

| *In vivo* angiogenesis (mice) - the number of new blood vessels following an subcutaneous introduction of the vectors | | | |
|---|---|---|---|
| | pVIF | pVEGF | pFGF |
| 3 days | 21,33 +/- 3,77 n= 15 | 14,50 +/- 2,26 n= 14 | 12,13 +/- 1,96 n= 15 |
| 13 days | 26,10 +/- 8,60 n=17 | 18,20 +/- 4,5 n= 15 | - |

### Example 5. Histochemical analysis

The mice used in the skin angiogenesis tests were used. Samples of subcutaneous tissue with the new blood vessels were taken. The purpose was to evaluate and compare the histological character of the blood vessels formed following the subcutaneous injection of the pVIF and pSEC/VEGF vectors. The results obtained prove that the pVIF vector induces the formation of more mature and larger blood vessels than the pSEC/VEGF vector.

### Example 6. Application of the pVIF vector in order to achieve angiogenesis in the cardiac muscle tissue of a patient with coronary disease.

The plasmid was used in a 52-year-old patient. The patient suffered from an unstable form of coronary disease of the ni/IV class according to the CCS, without a heart attack, with arterial hypertension, hypercholesterolemia and atherosclerosis in his lower limbs.

During the pre-operation stress test he achieved a 5,5 METS result. The test was interrupted due to intense changes in the ST region in the effluent vessels from the frontside and lower walls. The test result was described as singularly positive.

In the echocardiographic analysis, the effluent fraction was 65% (%EF=65). Coronography indicated multi-vessel coronary disease with a blocked circumflex artery (Cₓ), a blocked right coronary artery (RCA), thus it was only possible to bridge the left anterior descending artery (LAD).

During the operation, the left interior myocardial artery was grafted to the left anterior descending artery (LIMA to LAD) on a beating heart, without diverting blood externally. After performing the bridging to the coronary vessel, the bottom and side walls of the heart were injected with the pVIF plasmid solution. The cardiac muscle tissue was transfected directly, using an injection with a 0,33 mm needle.

No complications resulting from the gene transfection were noted, neither in the time during the operation, nor during the post-operational period. the patient was in a stable state throughout the time of the operation.

Presently, 3 months have passed since the operation, and the patient exhibits no athero-cardiac illnesses, and has functional breathing and circulation. In a SPECT analysis 2 weeks following the operation, perfusion was noted which was related to the isertion of the bridge to the LAD, and afterwards perfusion was noted in segments transfected with the pVIF plasmid in an examination performed 1 month following the operation. The patient's state is improving continually. In further examinations, revascularizing ischemic myocardial tissue was observed, which was exhibited by improvement of the vascular flow of the ischemic myocardium.

In summary, one may concluded that the application of the pVIF plasmid did not increase the risk of the operation. The patient bore the operation very well. No post-operational complications were observed. The patient does not report more coronary problems. His life comfort level has increased, along with his stress tolerance, and his requirement for nitrates has lessened.

## Claims

1. Expression box composed of a CMV promoter as well as an SV40 polyadenylation signal operationally connected to a gene complex containing:
a) a sequence coding a hybrid protein containing a secretory sequence stemming from immunoglobulin bound to the vascular endothelial growth factor 165 (VEGF₁₆₅) sequence with a removed native secretory sequence,
b) a sequence coding the hybrid protein containing a secretory sequence stemming from immunoglobulin bound to the fibroblast growth factor b (bFGF) sequence,
c) an Internal Ribosomal Entry Segment (IRES) sequence located between the mentioned sequences coding hybrid proteins,
wherein said secretory sequence stemming from immunoglobulin comprises amino acids residues 1-40 of SEQ ID No.: 1.

2. Expression box according to claim 1, **characterised in that** the sequence coding the hybrid protein mentioned in (a) is the coding sequence presented in Figure 5 or sequence presented as SEQ ID No.:1.

3. Expression box according to claim 1, **characterised in that** the sequence coding the hybrid protein mentioned in (b) is the coding sequence presented in Figure 2 or sequence presented as SEQ ID No.:3.

4. Expression box according to claim 1, **characterised in that** the IRES sequence mentioned in (c) is the sequence presented in Figure 4 or nucleotides 1348-1953 of SEQ ID No.:6.

5. Expression box according to claim 1, **characterised in that** it contains at least the nucleotides from 695 to 2538 of sequence presented as SEQ ID No.:5.

6. Two-cistron plasmid vector, **characterised in that** it contains an expression box according to claims 1-5.

7. The use of the expression box according to claims 1-5 for the production of an expression vector for use in gene therapy of ischemic states in humans.

8. A pharmaceutical composition for the treatment or prevention of ischemic disorders or diseases of the human being comprising an active ingredient and a pharmaceutically acceptable carrier or diluent **characterised in that** as the active ingredient it contains a two-cistron plasmid vector containing the expression box according to claims 1-5.

9. Use of the expression box according to claim 1 to 5 or vector according to claim 6 for the production of a medication for the treatment or prevention of ischemic disorders or diseases of the human being.

10. Use according to claim 9 wherein medication is produced for administering either in or near to site of the ischemic tissue.

11. Use according to claim 10 wherein ischemic tissue is an ischemic myocardium.

12. Use of the expression box according to claim 1 to 5 or vector according to claim 6 for the production of an medication for revascularizing ischemic tissue or improving the vascular flow of ischemic tissue.

13. Use according to claim 12 for production of a medication for administering either in or near to site of the ischemic tissue.

14. Use according to claim 12 wherein ischemic tissue is an ischemic myocardium.

## Patentansprüche

1. Expressionsbox, welche aus einem CMV-Promotor sowie einem SV40-Polyadenylierungssignal zusammengesetzt ist, und welche funktionell mit einem Genkomplex verbunden ist, welcher enthält:
(a) eine Sequenz, welche ein Hybridprotein codiert, wobei die Sequenz eine von einem Immunglobulin stammende sekretorische Sequenz enthält, welche mit der Sequenz des vaskulären endothelialen Wachstumsfaktors 165 (VEGF₁₆₅) verbunden ist, bei der die native sekretorische Sequenz entfernt ist,
(b) eine Sequenz, welche das Hybridprotein codiert, wobei die Sequenz eine von einem Immunglobulin stammende sekretorische Sequenz enthält, welche mit der Sequenz des Fibroblastenwachstumsfaktors b (bFGF) verbunden ist,
(c) eine Sequenz eines internen ribosomalen Eintrittsegments (IRES), welche zwischen den erwähnten Sequenzen, welche Hybridproteine codieren, lokalisiert ist,
wobei die von einem Immunglobulin stammende sekretorische Sequenz die Aminosäurereste 1 bis 40 von SEQ ID NO: 1 umfasst.

2. Expressionsbox nach Anspruch 1, **dadurch gekennzeichnet, dass** die Sequenz, welche das in (a) erwähnte Hybridprotein codiert, die codierende Sequenz wie in Figur 5 oder die Sequenz wie in SEQ ID NO: 1 gezeigt ist.

3. Expressionsbox nach Anspruch 1, **dadurch gekennzeichnet, dass** die Sequenz, welche das in (b) erwähnte Hybridprotein codiert, die codierende Sequenz wie in Figur 2 oder die Sequenz wie in SEQ ID NO: 3 gezeigt ist.

4. Expressionsbox nach Anspruch 1, **dadurch gekennzeichnet, dass** die in (c) erwähnte IRES-Sequenz die Sequenz wie in Figur 4 gezeigt ist oder die Nucleotide 1348 bis 1953 von SEQ ID NO: 6.

5. Expressionsbox nach Anspruch 1, **dadurch gekennzeichnet, dass** sie mindestens die Nucleotide von 695 bis 2538 der Sequenz wie in SEQ ID NO: 5 gezeigt enthält.

6. Bicistronischer Plasmidvektor, **dadurch gekennzeichnet, dass** er eine Expressionsbox nach einem der Ansprüche 1 bis 5 enthält.

7. Verwendung der Expressionsbox nach einem der Ansprüche 1 bis 5 für die Herstellung eines Expressionsvektors zur Verwendung in der Gentherapie von ischämischen Zuständen in Menschen.

8. Arzneimittel zur Behandlung oder Vorbeugung von ischämischen Störungen oder Krankheiten des Menschen, welches einen wirksamen Bestandteil und einen pharmazeutisch verträglichen Träger oder ein Verdünnungsmittel umfasst, wobei das Arzneimittel **dadurch gekennzeichnet ist, dass** es als wirksamen Bestandteil einen bicistronischen Plasmidvektor enthält, welcher die Expressionsbox nach einem der Ansprüche 1 bis 5 enthält.

9. Verwendung der Expressionsbox nach einem der Ansprüche 1 bis 5 oder des Vektors nach Anspruch 6 für die Herstellung eines Arzneimittels zur Behandlung oder Vorbeugung von ischämischen Störungen oder Krankheiten des Menschen.

10. Verwendung nach Anspruch 9, wobei das Arzneimittel hergestellt wird für die Verabreichung entweder in das ischämische Gewebe oder in die Nähe des ischämischen Gewebes.

11. Verwendung nach Anspruch 10, wobei das ischämische Gewebe ein ischämisches Myocard ist.

12. Verwendung der Expressionsbox nach einem der Ansprüche 1 bis 5 oder des Vektors nach Anspruch 6 für die Herstellung eines Arzneimittels zur Revaskularisation von ischämischem Gewebe oder zur Verbesserung des vaskulären Flusses in ischämischem Gewebe.

13. Verwendung nach Anspruch 12, für die Herstellung eines Arzneimittels für die Verarbreichung entweder in das ischämische Gewebe oder in die Nähe des ischämischen Gewebes.

14. Verwendung nach Anspruch 12, wobei das ischämische Gewebe ein ischämisches Myocard ist.

## Revendications

1. Boîte d'expression composée d'un promoteur CMV ainsi que d'un signal de polyadénylation du SV40 reliés de manière opérationnelle à un complexe génique contenant :
a) une séquence codant une protéine hybride contenant une séquence sécrétoire provenant de l'immunoglobuline liée à la séquence du facteur de croissance endothéliale vasculaire 165 (VBGF₁₆₅) avec une séquence sécrétoire native enlevée,
b) une séquence codant la protéine hybride contenant une séquence sécrétoire provenant de l'immunoglobuline liée à la séquence de facteur de croissance de fibroblaste b (bFGF),
c) une séquence de Segment d'Entrée Ribosomique Interne (IRES) située entre les séquences mentionnées codant les protéines hybrides,
dans laquelle ladite séquence sécrétoire provenant de l'immunoglobuline comprend des résidus d'acides aminés 1-40 de la SEQ ID N°1.

2. Boîte d'expression selon la revendication 1, **caractérisée en ce que** la séquence codant la protéine hybride mentionnée en (a) est la séquence codante présentée sur la figure 5 ou la séquence présentée comme SEQ ID N°1.

3. Boîte d'expression selon la revendication 1, **caractérisée en ce que** la séquence codant la protéine hybride mentionnée en (b) est la séquence codante présentée sur la figure 2 ou la séquence présentée comme SEQ ID N°3.

4. Boîte d'expression selon la revendication 1, **caractérisée en ce que** la séquence IRES mentionnée en (c) est la séquence présentée sur la figure 4 ou les nucléotides 1348-1353 de la SEQ ID N°6.

5. Boîte d'expression selon la revendication 1, **caractérisée en ce qu'**elle contient au moins les nucléotides de 695 à 2538 de la séquence présentée comme SEQ ID N°5.

6. Vecteur plasmide à deux cistrons, **caractérisé en ce qu'**il contient une boîte d'expression selon les revendications 1-5.

7. Utilisation de la boîte d'expression selon les revendications 1-5 pour la production d'un vecteur d'expression pour utiliser dans la thérapie génique d'états ischémiques chez l'homme.

8. Composition pharmaceutique pour le traitement ou la prévention de troubles ou maladies ischémiques de l'homme comprenant un ingrédient actif et un excipient ou diluant pharmaceutiquement acceptable, **caractérisée en ce que** l'ingrédient actif contient un vecteur plasmide à deux cistrons contenant la boîte d'expression selon les revendications 1-5.

9. Utilisation de la boîte d'expression selon les revendications 1 à 5 ou vecteur selon la revendication 6, pour la production d'un médicament pour le traitement ou la prévention de troubles ou maladies ischémiques de l'homme.

10. Utilisation selon la revendication 9, dans laquelle est produit un médicament à administrer dans ou près du site du tissu ischémique.

11. Utilisation selon la revendication 10, dans laquelle le tissu ischémique est un myocarde ischémique.

12. Utilisation de la boîte d'expression selon les revendications 1 à 5 ou vecteur selon la revendication 6, pour la production d'un médicament destiné à revasculariser le tissu ischémique ou à améliorer le flux vasculaire du tissu ischémique.

13. Utilisation selon la revendication 12, pour la production d'un médicament à administrer dans ou près du site du tissu ischémique.

14. Utilisation selon la revendication 12, dans laquelle le tissu ischémique est un myocarde ischémique.


### SEQUENCE LISTING

<110> ZAKEADY FARMACEUTYCZNE POLPHARMA S.A.
<120> EXPRESSION BOX, TWO-CISTRON PLASMID VECTOR, PHARMACEUTICAL AND THEIR USE IN ANGIOGENIC GENE THERAPY
<130> PZ/028/RW/PCT
<150> P 355255
   <151> 2002-08-05
<160> 6
<170> PatentIn version 3.1
<210> 1
   <211> 618
   <212> DNA
   <213> Artificial
<220>
   <221> CDS
   <222> (1)..(618)
   <223> ATG-Igk-VEGF
<400> 1
   atg gag aca gac aca ctc ctg cta tgg gta ctg ctg ctc tgg gtt cca       48
   Met Glu Thr Asp Thr Leu Leu Leu Trp Val Leu Leu Leu Trp Val Pro
   1               5                   10                  15
   ggt tcc act ggt gac gcg gcc cag ccg gcc agg cgc gcg cgc cgt acg       96
   Gly Ser Thr Gly Asp Ala Ala Gln Pro Ala Arg Arg Ala Arg Arg Thr
               20                  25                  30
   aag ctt ggt acc gag ctc gga tcc gca ccc atg gca gaa gga gga ggg      144
   Lys Leu Gly Thr Glu Leu Gly Ser Ala Pro Met Ala Glu Gly Gly Gly
           35                  40                  45
   cag aat cat cac gaa gtg gtg aag ttc atg gat gtc tat cag cgc agc      192
   Gln Asn His His Glu Val Val Lys Phe Met Asp Val Tyr Gln Arg Ser
       50                  55                  60
   tac tgc cat cca atc gag acc ctg gtg gac atc ttc cag gag tac cct      240
   Tyr Cys His Pro Ile Glu Thr Leu Val Asp Ile Phe Gln Glu Tyr Pro
   65                  70                  75                  80
   gat gag atc gag tac atc ttc aag cca tcc tgt gtg ccc ttg atg cga      288
   Asp Glu Ile Glu Tyr Ile Phe Lys Pro Ser Cys Val Pro Leu Met Arg
                   85                  90                  95
   tgc ggg ggc tgc tgc aat gac gag ggc ctg gag tgt gtg ccc act gag      336
   Cys Gly Gly Cys Cys Asn Asp Glu Gly Leu Glu Cys Val Pro Thr Glu
               100                 105                 110
   gag tcc aac att acc atg cag att atg cgg atc aaa cct cac caa ggc      384
   Glu Ser Asn Ile Thr Met Gln Ile Met Arg Ile Lys Pro His Gln Gly
           115                 120                 125
   cag cac ata gga gag atg agc ttc cta cag cac aac aaa tgt gaa tgc      432
   Gln His Ile Gly Glu Met Ser Phe Leu Gln His Asn Lys Cys Glu Cys
       130                 135                 140
   aga cca aag aaa gat aga gca aga caa gaa aat ccc tgt ggg cct tgc      480
   Arg Pro Lys Lys Asp Arg Ala Arg Gln Glu Asn Pro Cys Gly Pro Cys
   145                 150                 155                 160
   tca gag cgg aga aag cat ttg ttt gta caa gat ccg cag acg tgt aaa      528
   Ser Glu Arg Arg Lys His Leu Phe Val Gln Asp Pro Gln Thr Cys Lys
                   165                 170                 175
   tgt tcc tgc aaa aac aca gac tcg agt tgc aag gcg agg cag ctt gag      576
   Cys Ser Cys Lys Asn Thr Asp Ser Ser Cys Lys Ala Arg Gln Leu Glu
               180                 185                 190
   tta aac gaa cgt act tgc aga tgt gac aag ccg agg cgg tga              618
   Leu Asn Glu Arg Thr Cys Arg Cys Asp Lys Pro Arg Arg
           195                 200                 205
<210> 2
   <211> 205
   <212> PRT
   <213> Artificial
<400> 2
   Met Glu Thr Asp Thr Leu Leu Leu Trp Val Leu Leu Leu Trp Val Pro
   1               5                   10                  15
   Gly Ser Thr Gly Asp Ala Ala Gln Pro Ala Arg Arg Ala Arg Arg Thr
   Lys Leu Gly Thr Glu Leu Gly Ser Ala Pro Met Ala Glu Gly Gly Gly
   35 40 45
   Gln Asn His His Glu Val Val Lys Phe Met Asp Val Tyr Gln Arg Ser
   50 55 60
   Tyr Cys His Pro Ile Glu Thr Leu Val Asp Ile Phe Gln Glu Tyr Pro
   65 70 75 80
   Asp Glu Ile Glu Tyr Ile Phe Lys Pro Ser Cys Val Pro Leu Met Arg
   85 90 95
   Cys Gly Gly Cys Cys Asn Asp Glu Gly Leu Glu Cys Val Pro Thr Glu
   100 105 110
   Glu Ser Asn Ile Thr Met Gln Ile Met Arg Ile Lys Pro His Gln Gly
   115 120 125
   Gln His Ile Gly Glu Met Ser Phe Leu Gln His Asn Lys Cys Glu Cys
   130 135 140
   Arg Pro Lys Lys Asp Arg Ala Arg Gln Glu Asn Pro Cys Gly Pro Cys
   145 150 155 160
   Ser Glu Arg Arg Lys His Leu Phe Val Gln Asp Pro Gln Thr Cys Lys
   165 170 175
   Cys Ser Cys Lys Asn Thr Asp Ser Ser Cys Lys Ala Arg Gln Leu Glu
   180 185 190
   Leu Asn Glu Arg Thr Cys Arg Cys Asp Lys Pro Arg Arg
   195 200 205
<210> 3
   <211> 582
   <212> DNA
   <213> Artificial
<220>
   <221> CDS
   <222> (1)..(582)
   <223> ATG-Igk-bFGF
<400> 3
   atg gag aca gac aca ctc ctg cta tgg gta ctg ctg ctc tgg gtt cca      48
   Met Glu Thr Asp Thr Leu Leu Leu Trp Val Leu Leu Leu Trp Val Pro
   1               5                   10                  15
   ggt tcc act ggt gac gcg gcc cag ccg gcc agg cgc gcg cgc cgt acg      96
   Gly Ser Thr Gly Asp Ala Ala Gln Pro Ala Arg Arg Ala Arg Arg Thr
               20                  25                  30
   aag ctt ggt acc gag ctc gga tcc act cca gtg tgg tgg aat tcg cca     144
   Lys Leu Gly Thr Glu Leu Gly Ser Thr Pro Val Trp Trp Asn Ser Pro
           35                  40                  45
   gca ttg ccc gag gat ggc ggc agc ggc gcc ttc ccg ccc ggc cac ttc     192
   Ala Leu Pro Glu Asp Gly Gly Ser Gly Ala Phe Pro Pro Gly His Phe
       50                  55                  60
   aag gac ccc aag cgg ctg tac tgc aaa aac ggg ggc ttc ttc ctg cgc     240
   Lys Asp Pro Lys Arg Leu Tyr Cys Lys Asn Gly Gly Phe Phe Leu Arg
   65                  70                  75                  80
   atc cac ccc gac ggc cga gtt gac ggg gtc cgg gag aag agc gac cct     288
   Ile His Pro Asp Gly Arg Val Asp Gly Val Arg Glu Lys Ser Asp Pro
                   85                  90                  95
   cac atc aag cta caa ctt caa gca gaa gag aga gga gtt gtg tct atc     336
   His Ile Lys Leu Gln Leu Gln Ala Glu Glu Arg Gly Val Val Ser Ile
               100                 105                 110
   aaa gga gtg tgt gct aac cgt tac ctg gct atg aag gaa gat gga aga     384
   Lys Gly Val Cys Ala Asn Arg Tyr Leu Ala Met Lys Glu Asp Gly Arg
           115                 120                 125
   tta ctg gct tct aaa tgt gtt acg gat gag tgt ttc ttt ttt gaa cga     432
   Leu Leu Ala Ser Lys Cys Val Thr Asp Glu Cys Phe Phe Phe Glu Arg
       130                 135                 140
   ttg gaa tct aat aac tac aat act tac cgg tca agg aaa tac acc agt     480
   Leu Glu Ser Asn Asn Tyr Asn Thr Tyr Arg Ser Arg Lys Tyr Thr Ser
   145                 150                 155                 160
   tgg tat gtg gca ctg aaa cga act ggg cag tat aaa ctt gga tcc aaa     528
   Trp Tyr Val Ala Leu Lys Arg Thr Gly Gln Tyr Lys Leu Gly Ser Lys
                   165                 170                 175
   aca gga cct ggg cag aaa gct ata ctt ttt ctt cca atg tct gct aag     576
   Thr Gly Pro Gly Gln Lys Ala Ile Leu Phe Leu Pro Met Ser Ala Lys
               180                 185                 190
   agc tga                                                             582
   Ser
<210> 4
   <211> 193
   <212> PRT
   <213> Artificial
<400> 4
   Met Glu Thr Asp Thr Leu Leu Leu Trp Val Leu Leu Leu Trp Val Pro
   1               5                   10                  15
   Gly Ser Thr Gly Asp Ala Ala Gln Pro Ala Arg Arg Ala Arg Arg Thr
               20                  25                  30
   Lys Leu Gly Thr Glu Leu Gly Ser Thr Pro Val Trp Trp Asn Ser Pro
           35                  40                  45
   Ala Leu Pro Glu Asp Gly Gly Ser Gly Ala Phe Pro Pro Gly His Phe
       50                  55                  60
   Lys Asp Pro Lys Arg Leu Tyr Cys Lys Asn Gly Gly Phe Phe Leu Arg
   65                  70                  75                  80
   Ile His Pro Asp Gly Arg Val Asp Gly Val Arg Glu Lys Ser Asp Pro
                   85                  90                  95
   His Ile Lys Leu Gln Leu Gln Ala Glu Glu Arg Gly Val Val Ser Ile
               100                 105                 110
   Lys Gly Val Cys Ala Asn Arg Tyr Leu Ala Met Lys Glu Asp Gly Arg
           115                 120                 125
   Leu Leu Ala Ser Lys Cys Val Thr Asp Glu Cys Phe Phe Phe Glu Arg
       130                 135                 140
   Leu Glu Ser Asn Asn Tyr Asn Thr Tyr Arg Ser Arg Lys Tyr Thr Ser
   145                 150                 155                 160
   Trp Tyr Val Ala Leu Lys Arg Thr Gly Gln Tyr Lys Leu Gly Ser Lys
                   165                 170                 175
   Thr Gly Pro Gly Gln Lys Ala Ile Leu Phe Leu Pro Met Ser Ala Lys
               180                 185                 190
   Ser
<210> 5
   <211> 2538
   <212> DNA
   <213> Artificial
<220> .
   <221> gene
   <222> (695)..(2538)
   <223> casette
<400> 5
   gatgtacggg ccagatatac gcgttgacat tgattattga ctagttatta atagtaatca      60
   attacggggt cattagttca tagcccatat atggagttcc gcgttacata acttacggta     120
   aatggcccgc ctggctgacc gcccaacgcc cccgcccatt gacgtcaata atgacgtatg     180
   ttcccatagt aacgccaata gggactttcc attgacgtca atgggtggac tatttacggt     240
   aaactgccca cttggcagta catcaagtgt atcatatgcc aagtacgccc cctattgacg     300
   tcaatgacgg taaatggccc gcctggcatt atgcccagta catgacctta tgggactttc     360
   ctacttggca gtacatctac gtattagtca tcgctattac catggtgatg cggttttggc     420
   agtacatcaa tgggcgtgga tagcggtttg actcacgggg atttccaagt ctccacccca     480
   ttgacgtcaa tgggagtttg ttttggcacc aaaatcaacg ggactttcca aaatgtcgta     540
   acaactccgc cccattgacg caaatgggcg gtaggcgtgt acggtgggag gtctatataa     600
   gcagagctct ctggctaact agagaaccca ctgcttactg gcttatcgaa attaatacga     660
   ctcactatag ggagacccaa gctggctagc caccatggag acagacacac tcctgctatg     720
   ggtactgctg ctctgggttc caggttccac tggtgacgcg gcccagccgg ccaggcgcgc     780
   gcgccgtacg aagcttggta ccgagctcgg atccgcaccc atggcagaag gaggagggca     840
   gaatcatcac gaagtggtga agttcatgga tgtctatcag cgcagctact gccatccaat     900
   cgagaccctg gtggacatct tccaggagta ccctgatgag atcgagtaca tcttcaagcc     960
   cctgtgtgcc cttgatgcga tgcgggggct gctgcaatga cgagggcctg gagtgtgtgc    1020
   ccactgagga gtccaacatt accatgcaga ttatgcggat caaacctcac caaggccagc    1080
   acataggaga gatgagcttc ctacagcaca acaaatgtga atgcagacca aagaaagata    1140
   gagcaagaca agaaaatccc tgtgggcctt gctcagagcg gagaaagcat ttgtttgtac    1200
   aagatccgca gacgtgtaaa tgttcctgca aaaacacaga ctcgagttgc aaggcgaggc    1260
   agcttgagtt aaacgaacgt acttgcagat gtgacaagcc gaggcggtga gccgggcagg 1320
   aggaaggagc ctccctcagc gtttcgggaa ttccaattcc gcccctctcc ctcccccccc 1380
   cctaacgtta ctggccgaag ccgcttggaa taaggccggt gtgcgtttgt ctatatgtga 1440
   ttttccacca tattgccgtc tttggcaatg tgagggcccg gaaacctggc cctgtcttct 1500
   tgacgagcat tcctaggggt ctttcccctc tcgccaaagg aatgcaaggt ctgttgaatg 1560
   tcgtgaagga agcagttcct ctggaagctt cttgaagaca aacaacgtct gtagcgaccc 1620
   tttgcaggca gcggaacccc ccacctggcg acaggtgcct ctgcggccaa aagccacgtg 1680
   tataagatac acctgcaaag gcggcacaac cccagtgcca cgttgtgagt tggatagttg 1740
   tggaaagagt caaatggctc tcctcaagcg tattcaacaa ggggctgaag gatgcccaga 1800
   aggtacccca ttgtatggga tctgatctgg ggcctcggtg cacatgcttt acatgtgttt 1860
   agtcgaggtt aaaaaaacgt ctaggccccc cgaaccacgg ggacgtggtt ttcctttgaa 1920
   aaacacgatg ataagcttgc cacaagcggc cgcaccatgg agacagacac actcctgcta 1980
   tgggtactgc tgctctgggt tccaggttcc actggtgacg cggcccagcc ggccaggcgc 2040
   gcgcgccgta cgaagcttgg taccgagctc ggatccactc cagtgtggtg gaattcgcca 2100
   gcattgcccg aggatggcgg cagcggcgcc ttcccgcccg gccacttcaa ggaccccaag 2160
   cggctgtact gcaaaaacgg gggcttcttc ctgcgcatcc accccgacgg ccgagttgac 2220
   ggggtccggg agaagagcga ccctcacatc aagctacaac ttcaagcaga agagagagga 2280
   gttgtgtcta tcaaaggagt gtgtgctaac cgttacctgg ctatgaagga agatggaaga 2340
   ttactggctt ctaaatgtgt tacggatgag tgtttctttt ttgaacgatt ggaatctaat 2400
   aactacaata cttaccggtc aaggaaatac accagttggt atgtggcact gaaacgaact 2460
   gggcagtata aacttggatc caaaacagga cctgggcaga aagctatact tttcttccca 2520
   atgtctgcta agagctga 2538
<210> 6
   <211> 2537
   <212> DNA
   <213> Encephalomyocarditis virus
<220>
   <221> IRES
   <222> (1)..(2537)
   <223>
<400> 6
   gatgtacggg ccagatatac gcgttgacat tgattattga ctagttatta atagtaatca 60
   attacggggt cattagttca tagcccatat atggagttcc gcgttacata acttacggta 120
   aatggcccgc ctggctgacc gcccaacgcc cccgcccatt gacgtcaata atgacgtatg 180
   ttcccatagt aacgccaata gggactttcc attgacgtca atgggtggac tatttacggt 240
   aaactgccca cttggcagta catcaagtgt atcatatgcc aagtacgccc cctattgacg 300
   tcaatgacgg taaatggccc gcctggcatt atgcccagta catgacctta tgggactttc 360
   ctacttggca gtacatctac gtattagtca tcgctattac catggtgatg cggttttggc 420
   agtacatcaa tgggcgtgga tagcggtttg actcacgggg atttccaagt ctccacccca 480
   ttgacgtcaa tgggagtttg ttttggcacc aaaatcaacg ggactttcca aaatgtcgta 540
   acaactccgc cccattgacg caaatgggcg gtaggcgtgt acggtgggag gtctatataa 600
   gcagagctct ctggctaact agagaaccca ctgcttactg gcttatcgaa attaatacga 660
   ctcactatag ggagacccaa gctggctagc caccatggag acagacacac tcctgctatg 720
   ggtactgctg ctctgggttc caggttccac tggtgacgcg gcccagccgg ccaggcgcgc 780
   gcgccgtacg aagcttggta ccgagctcgg atccgcaccc atggcagaag gaggagggca 840
   gaatcatcac gaagtggtga agttcatgga tgtctatcag cgcagctact gccatccaat 900
   cgagaccctg gtggacatct tccaggagta ccctgatgag atcgagtaca tcttcaagcc 960
   cctgtgtgcc cttgatgcga tgcgggggct gctgcaatga cgagggcctg gagtgtgtgc 1020
   ccactgagga gtccaacatt accatgcaga ttatgcggat caaacctcac caaggccagc 1080
   acataggaga gatgagcttc ctacagcaca acaaatgtga atgcagacca aagaaagata 1140
   gagcaagaca agaaaatccc tgtgggcctt gctcagagcg gagaaagcat ttgtttgtac 1200
   aagatccgca gacgtgtaaa tgttcctgca aaaacacaga ctcgagttgc aaggcgaggc 1260
   agcttgagtt aaacgaacgt acttgcagat gtgacaagcc gaggcggtga gccgggcagg 1320
   aggaaggagc ctccctcagc gtttcgggaa ttccaattcc gcccctctcc ctcccccccc 1380
   cctaacgtta ctggccgaag ccgcttggaa taaggccggt gtgcgtttgt ctatatgtga 1440
   ttttccacca tattgccgtc tttggcaatg tgagggcccg gaaacctggc cctgtcttct 1500
   tgacgagcat tcctaggggt ctttcccctc tcgccaaagg aatgcaaggt ctgttgaatg 1560
   tcgtgaagga agcagttcct ctggaagctt cttgaagaca aacaacgtct gtagcgaccc 1620
   tttgcaggca gcggaacccc ccacctggcg acaggtgcct ctgcggccaa aagccacgtg 1680
   tataagatac acctgcaaag gcggcacaac cccagtgcca cgttgtgagt tggatagttg   1740
   tggaaagagt caaatggctc tcctcaagcg tattcaacaa ggggctgaag gatgcccaga   1800
   aggtacccca ttgtatggga tctgatctgg ggcctcggtg cacatgcttt acatgtgttt   1860
   agtcgaggtt aaaaaaacgt ctaggccccc cgaaccacgg ggacgtggtt ttcctttgaa   1920
   aaacacgatg ataagcttgc cacaagcggc cgcaccatgg agacagacac actcctgcta   1980
   tgggtactgc tgctctgggt tccaggttcc actggtgacg cggcccagcc ggccaggcgc   2040
   gcgcgccgta cgaagcttgg taccgagctc ggatccactc cagtgtggtg gaattcgcca   2100
   gcattgcccg aggatggcgg cagcggcgct tcccgcccgg ccacttcaag gaccccaagc   2160
   ggctgtactg caaaaacggg ggcttcttcc tgcgcatcca ccccgacggc cgagttgacg   2220
   gggtccggga gaagagcgac cctcacatca agctacaact tcaagcagaa gagagaggag   2280
   ttgtgtctat caaaggagtg tgtgctaacc gttacctggc tatgaaggaa gatggaagat   2340
   tactggcttc taaatgtgtt acggatgagt gtttcttttt tgaacgattg gaatctaata   2400
   actacaatac ttaccggtca aggaaataca ccagttggta tgtggcactg aaacgaactg   2460
   ggcagtataa acttggatcc aaaacaggac ctgggcagaa agctatactt tttcttccaa   2520
   tgtctgctaa gagctga                                                  2537

### SEQUENCE LISTING

<110> ZAKLADY FARMACEUTYCZNE POLPHARMA S.A.
<120> EXPRESSION BOX, TWO-CISTRON PLASMID VECTOR, PHARMACEUTICAL AND THEIR USE IN ANGIOGENIC GENE THERAPY
<130> PZ/028/RW/PCT
<150> P 355255
   <151> 2002-08-05
<160> 6
<170> PatentIn version 3.2
<210> 1
   <211> 618
   <212> DNA
   <213> Artificial
<220>
   <223> Igk-VEGF
<220>
   <221> CDS
   <222> (1)..(618)
   <223> ATG-Igk-VEGF
<400> 1
   atg gag aca gac aca ctc ctg cta tgg gta ctg ctg ctc tgg gtt cca        48
   Met Glu Thr Asp Thr Leu Leu Leu Trp Val Leu Leu Leu Trp Val Pro
   1               5                   10                  15
   ggt tcc act ggt gac gcg gcc cag ccg gcc agg cgc gcg cgc cgt acg        96
   Gly Ser Thr Gly Asp Ala Ala Gln Pro Ala Arg Arg Ala Arg Arg Thr
               20                  25                  30
   aag ctt ggt acc gag ctc gga tcc gca ccc atg gca gaa gga gga ggg       144
   Lys Leu Gly Thr Glu Leu Gly Ser Ala Pro Met Ala Glu Gly Gly Gly
           35                  40                  45
   cag aat cat cac gaa gtg gtg aag ttc atg gat gtc tat cag cgc agc       192
   Gln Asn His His Glu Val Val Lys Phe Met Asp Val Tyr Gln Arg Ser
       50                  55                  60
   tac tgc cat cca atc gag acc ctg gtg gac atc ttc cag gag tac cct       240
   Tyr Cys His Pro Ile Glu Thr Leu Val Asp Ile Phe Gln Glu Tyr Pro
   65                  70                  75                  80
   gat gag atc gag tac atc ttc aag cca tcc tgt gtg ccc ttg atg cga       288
   Asp Glu Ile Glu Tyr Ile Phe Lys Pro Ser Cys Val Pro Leu Met Arg
                   85                  90                  95
   tgc ggg ggc tgc tgc aat gac gag ggc ctg gag tgt gtg ccc act gag       336
   Cys Gly Gly Cys Cys Asn Asp Glu Gly Leu Glu Cys Val Pro Thr Glu
               100                 105                 110
   gag tcc aac att acc atg cag att atg cgg atc aaa cct cac caa ggc       384
   Glu Ser Asn Ile Thr Met Gln Ile Met Arg Ile Lys Pro His Gln Gly
           115                 120                 125
   cag cac ata gga gag atg agc ttc cta cag cac aac aaa tgt gaa tgc       432
   Gln His Ile Gly Glu Met Ser Phe Leu Gln His Asn Lys Cys Glu Cys
       130                 135                 140
   aga cca aag aaa gat aga gca aga caa gaa aat ccc tgt ggg cct tgc       480
   Arg Pro Lys Lys Asp Arg Ala Arg Gln Glu Asn Pro Cys Gly Pro Cys
   145                 150                 155                 160
   tca gag cgg aga aag cat ttg ttt gta caa gat ccg cag acg tgt aaa       528
   Ser Glu Arg Arg Lys His Leu Phe Val Gln Asp Pro Gln Thr Cys Lys
                   165                 170                 175
   tgt tcc tgc aaa aac aca gac tcg agt tgc aag gcg agg cag ctt gag       576
   Cys Ser Cys Lys Asn Thr Asp Ser Ser Cys Lys Ala Arg Gln Leu Glu
               180                 185                 190
   tta aac gaa cgt act tgc aga tgt gac aag ccg agg cgg tga               618
   Leu Asn Glu Arg Thr Cys Arg Cys Asp Lys Pro Arg Arg
           195                 200                 205
<210> 2
   <211> 205
   <212> PRT
   <213> Artificial
<220>
   <223> Synthetic Construct
<400> 2
   Met Glu Thr Asp Thr Leu Leu Leu Trp Val Leu Leu Leu Trp Val Pro
   1               5                   10                  15
   Gly Ser Thr Gly Asp Ala Ala Gln Pro Ala Arg Arg Ala Arg Arg Thr
               20                  25                  30
   Lys Leu Gly Thr Glu Leu Gly Ser Ala Pro Met Ala Glu Gly Gly Gly
           35                  40                  45
   Gln Asn His His Glu Val Val Lys Phe Met Asp Val Tyr Gln Arg Ser
       50                  55                  60
   Tyr Cys His Pro Ile Glu Thr Leu Val Asp Ile Phe Gln Glu Tyr Pro
   65                  70                  75                  80
   Asp Glu Ile Glu Tyr Ile Phe Lys Pro Ser Cys Val Pro Leu Met Arg
                   85                  90                  95
   Cys Gly Gly Cys Cys Asn Asp Glu Gly Leu Glu Cys Val Pro Thr Glu
               100                 105                 110
   Glu Ser Asn Ile Thr Met Gln Ile Met Arg Ile Lys Pro His Gln Gly
           115                 120                 125
   Gln His Ile Gly Glu Met Ser Phe Leu Gln His Asn Lys Cys Glu Cys
       130                 135                 140
   Arg Pro Lys Lys Asp Arg Ala Arg Gln Glu Asn Pro Cys Gly Pro Cys
   145                 150                 155                 160
   Ser Glu Arg Arg Lys His Leu Phe Val Gln Asp Pro Gln Thr Cys Lys
                   165                 170                 175
   Cys Ser Cys Lys Asn Thr Asp Ser Ser Cys Lys Ala Arg Gln Leu Glu
               180                 185                 190
   Leu Asn Glu Arg Thr Cys Arg Cys Asp Lys Pro Arg Arg
           195                 200                 205
<210> 3
   <211> 582
   <212> DNA
   <213> Artificial
<220>
   <223> Igk-bFGF
<220>
   <221> CDS
   <222> (1)..(582)
   <223> ATG-Igk-bFGF
<400> 3
   atg gag aca gac aca ctc ctg cta tgg gta ctg ctg ctc tgg gtt cca        48
   Met Glu Thr Asp Thr Leu Leu Leu Trp Val Leu Leu Leu Trp Val Pro
   1               5                   10                  15
   ggt tcc act ggt gac gcg gcc cag ccg gcc agg cgc gcg cgc cgt acg        96
   Gly Ser Thr Gly Asp Ala Ala Gln Pro Ala Arg Arg Ala Arg Arg Thr
               20                  25                  30
   aag ctt ggt acc gag ctc gga tcc act cca gtg tgg tgg aat tcg cca       144
   Lys Leu Gly Thr Glu Leu Gly Ser Thr Pro Val Trp Trp Asn Ser Pro
           35                  40                  45
   gca ttg ccc gag gat ggc ggc agc ggc gcc ttc ccg ccc ggc cac ttc       192
   Ala Leu Pro Glu Asp Gly Gly Ser Gly Ala Phe Pro Pro Gly His Phe
       50                  55                  60
   aag gac ccc aag cgg ctg tac tgc aaa aac ggg ggc ttc ttc ctg cgc       240
   Lys Asp Pro Lys Arg Leu Tyr Cys Lys Asn Gly Gly Phe Phe Leu Arg
   65                  70                  75                  80
   atc cac ccc gac ggc cga gtt gac ggg gtc cgg gag aag agc gac cct       288
   Ile His Pro Asp Gly Arg Val Asp Gly Val Arg Glu Lys Ser Asp Pro
                   85                  90                  95
   cac atc aag cta caa ctt caa gca gaa gag aga gga gtt gtg tct atc       336
   His Ile Lys Leu Gln Leu Gln Ala Glu Glu Arg Gly Val Val Ser Ile
               100                 105                 110
   aaa gga gtg tgt gct aac cgt tac ctg gct atg aag gaa gat gga aga       384
   Lys Gly Val Cys Ala Asn Arg Tyr Leu Ala Met Lys Glu Asp Gly Arg
           115                 120                 125
   tta ctg gct tct aaa tgt gtt acg gat gag tgt ttc ttt ttt gaa cga       432
   Leu Leu Ala Ser Lys Cys Val Thr Asp Glu Cys Phe Phe Phe Glu Arg
       130                 135                 140
   ttg gaa tct aat aac tac aat act tac cgg tca agg aaa tac acc agt       480
   Leu Glu Ser Asn Asn Tyr Asn Thr Tyr Arg Ser Arg Lys Tyr Thr Ser
   145                 150                 155                 160
   tgg tat gtg gca ctg aaa cga act ggg cag tat aaa ctt gga tcc aaa       528
   Trp Tyr Val Ala Leu Lys Arg Thr Gly Gln Tyr Lys Leu Gly Ser Lys
                   165                 170                 175
   aca gga cct ggg cag aaa gct ata ctt ttt ctt cca atg tct gct aag       576
   Thr Gly Pro Gly Gln Lys Ala Ile Leu Phe Leu Pro Met Ser Ala Lys
               180                 185                 190
   agc tga                                                               582
   Ser
<210> 4
   <211> 193
   <212> PRT
   <213> Artificial
<220>
   <223> Synthetic Construct
<400> 4
   Met Glu Thr Asp Thr Leu Leu Leu Trp Val Leu Leu Leu Trp Val Pro
   1               5                   10                  15
   Gly Ser Thr Gly Asp Ala Ala Gln Pro Ala Arg Arg Ala Arg Arg Thr
               20                  25                  30
   Lys Leu Gly Thr Glu Leu Gly Ser Thr Pro Val Trp Trp Asn Ser Pro
           35                  40                  45
   Ala Leu Pro Glu Asp Gly Gly Ser Gly Ala Phe Pro Pro Gly His Phe
       50                  55                  60
   Lys Asp Pro Lys Arg Leu Tyr Cys Lys Asn Gly Gly Phe Phe Leu Arg
   65                  70                  75                  80
   Ile His Pro Asp Gly Arg Val Asp Gly Val Arg Glu Lys Ser Asp Pro
                   85                  90                  95
   His Ile Lys Leu Gln Leu Gln Ala Glu Glu Arg Gly Val Val Ser Ile
               100                 105                 110
   Lys Gly Val Cys Ala Asn Arg Tyr Leu Ala Met Lys Glu Asp Gly Arg
           115                 120                 125
   Leu Leu Ala Ser Lys Cys Val Thr Asp Glu Cys Phe Phe Phe Glu Arg
       130                 135                 140
   Leu Glu Ser Asn Asn Tyr Asn Thr Tyr Arg Ser Arg Lys Tyr Thr Ser
   145                 150                 155                 160
   Trp Tyr Val Ala Leu Lys Arg Thr Gly Gln Tyr Lys Leu Gly Ser Lys
                   165                 170                 175
   Thr Gly Pro Gly Gln Lys Ala Ile Leu Phe Leu Pro Met Ser Ala Lys
               180                 185                 190
   Ser
<210> 5
   <211> 2538
   <212> DNA
   <213> Artificial
<220>
   <223> casette CMV-ATG-Igk-VEGF-TGA-IRES-ATG-Igk-bFGF-TGA
<220>
   <221> gene
   <222> (695)..(2538)
   <223> casette
<400> 5
   gatgtacggg ccagatatac gcgttgacat tgattattga ctagttatta atagtaatca      60
   attacggggt cattagttca tagcccatat atggagttcc gcgttacata acttacggta     120
   aatggcccgc ctggctgacc gcccaacgcc cccgcccatt gacgtcaata atgacgtatg     180
   ttcccatagt aacgccaata gggactttcc attgacgtca atgggtggac tatttacggt     240
   aaactgccca cttggcagta catcaagtgt atcatatgcc aagtacgccc cctattgacg     300
   tcaatgacgg taaatggccc gcctggcatt atgcccagta catgacctta tgggactttc     360
   ctacttggca gtacatctac gtattagtca tcgctattac catggtgatg cggttttggc     420
   agtacatcaa tgggcgtgga tagcggtttg actcacgggg atttccaagt ctccacccca     480
   ttgacgtcaa tgggagtttg ttttggcacc aaaatcaacg ggactttcca aaatgtcgta     540
   acaactccgc cccattgacg caaatgggcg gtaggcgtgt acggtgggag gtctatataa     600
   gcagagctct ctggctaact agagaaccca ctgcttactg gcttatcgaa attaatacga     660
   ctcactatag ggagacccaa gctggctagc caccatggag acagacacac tcctgctatg     720
   ggtactgctg ctctgggttc caggttccac tggtgacgcg gcccagccgg ccaggcgcgc     780
   gcgccgtacg aagcttggta ccgagctcgg atccgcaccc atggcagaag gaggagggca     840
   gaatcatcac gaagtggtga agttcatgga tgtctatcag cgcagctact gccatccaat     900
   cgagaccctg gtggacatct tccaggagta ccctgatgag atcgagtaca tcttcaagcc     960
   cctgtgtgcc cttgatgcga tgcgggggct gctgcaatga cgagggcctg gagtgtgtgc    1020
   ccactgagga gtccaacatt accatgcaga ttatgcggat caaacctcac caaggccagc    1080
   acataggaga gatgagcttc ctacagcaca acaaatgtga atgcagacca aagaaagata    1140
   gagcaagaca agaaaatccc tgtgggcctt gctcagagcg gagaaagcat ttgtttgtac    1200
   aagatccgca gacgtgtaaa tgttcctgca aaaacacaga ctcgagttgc aaggcgaggc    1260
   agcttgagtt aaacgaacgt acttgcagat gtgacaagcc gaggcggtga gccgggcagg    1320
   aggaaggagc ctccctcagc gtttcgggaa ttccaattcc gcccctctcc ctcccccccc    1380
   cctaacgtta ctggccgaag ccgcttggaa taaggccggt gtgcgtttgt ctatatgtga    1440
   ttttccacca tattgccgtc tttggcaatg tgagggcccg gaaacctggc cctgtcttct    1500
   tgacgagcat tcctaggggt ctttcccctc tcgccaaagg aatgcaaggt ctgttgaatg    1560
   tcgtgaagga agcagttcct ctggaagctt cttgaagaca aacaacgtct gtagcgaccc   1620
   tttgcaggca gcggaacccc ccacctggcg acaggtgcct ctgcggccaa aagccacgtg   1680
   tataagatac acctgcaaag gcggcacaac cccagtgcca cgttgtgagt tggatagttg   1740
   tggaaagagt caaatggctc tcctcaagcg tattcaacaa ggggctgaag gatgcccaga   1800
   aggtacccca ttgtatggga tctgatctgg ggcctcggtg cacatgcttt acatgtgttt   1860
   agtcgaggtt aaaaaaacgt ctaggccccc cgaaccacgg ggacgtggtt ttcctttgaa   1920
   aaacacgatg ataagcttgc cacaagcggc cgcaccatgg agacagacac actcctgcta   1980
   tgggtactgc tgctctgggt tccaggttcc actggtgacg cggcccagcc ggccaggcgc   2040
   gcgcgccgta cgaagcttgg taccgagctc ggatccactc cagtgtggtg gaattcgcca   2100
   gcattgcccg aggatggcgg cagcggcgcc ttcccgcccg gccacttcaa ggaccccaag   2160
   cggctgtact gcaaaaacgg gggcttcttc ctgcgcatcc accccgacgg ccgagttgac   2220
   ggggtccggg agaagagcga ccctcacatc aagctacaac ttcaagcaga agagagagga   2280
   gttgtgtcta tcaaaggagt gtgtgctaac cgttacctgg ctatgaagga agatggaaga   2340
   ttactggctt ctaaatgtgt tacggatgag tgtttctttt ttgaacgatt ggaatctaat   2400
   aactacaata cttaccggtc aaggaaatac accagttggt atgtggcact gaaacgaact   2460
   gggcagtata aacttggatc caaaacagga cctgggcaga aagctatact ttttcttcca   2520
   atgtctgcta agagctga                                                 2538
<210> 6
   <211> 2537
   <212> DNA
   <213> Encephalomyocarditis virus
<220>
   <221> IRES
   <222> (1)..(2537)
<400> 6